# EUROPEAN PATENT SPECIFICATION

(11) **EP 4 551 950 B1**
(45) Date of publication and mention of the grant of the patent: **02.12.2026**
(21) Application number: 23834346.1
(22) Date of filing: 10.07.2023
(51) Int. Cl.: G01P 5/22, G01P 5/20, G01P 5/26, G01P 13/04, A61B 5/026, A61B 5/00, G01F 1/661

(54) **VELOCITY MEASUREMENT BY DECORRELATION RATIO OF STRUCTURED OPTICAL SIGNALS**
GESCHWINDIGKEITSMESSUNG DURCH DEKORRELATIONSVERHÄLTNIS VON STRUKTURIERTEN OPTISCHEN SIGNALEN
MESURE DE VITESSE PAR RAPPORT DE DÉCORRÉLATION DE SIGNAUX OPTIQUES STRUCTURÉS

(30) Priority: 08.07.2022 US 202263359665 P
(43) Date of publication of application: 14.05.2025
(73) Proprietor: École Polytechnique De Montréal, Montréal Québec H3T 1J4 (CA)
(72) Inventor: BOUDOUX, Caroline, Montréal, Québec H1Y 3K4 (CA); MALTAIS-TARIANT, Raphaël, Montréal, Québec H2R 1Z2 (CA)
(74) Representative: Regimbeau
(86) International application number: PCT/CA2023/050926
(87) International publication number: WO 2024/007087

(56) References cited:
- WO-A1-2021/087333
- WO-A1-2021/151194
- US-A1- 2006 132 790
- US-A1- 2010 166 293
- US-A1- 2011 149 291
- US-A1- 2012 188 555
- US-A1- 2012 300 215
- US-A1- 2015 101 411

## Description

### CROSS-REFERENCE TO RELATED APPLICATIONS

The present application claims priority on United States Patent Application No. 63/359,665 filed July 8, 2022.

### FIELD

The present disclosure generally relates to the field of velocity measurement, and more specifically to velocity measurement by decorrelation of structured electromagnetic signals.

### BACKGROUND

The estimation of a flow of particles, such as the blood flow in a vessel, can be a useful diagnostic indicator in a number of diseases, in particular retinopathies. Although obtaining an accurate estimate of blood flow is an important consideration in the study and follow-up of disease progression, this information may be very difficult, if not impossible, to obtain in a non-invasive and accurate manner. Blood vessels of interest in the retina of the eye may be very small and are often positioned inconveniently, limiting the methods by which the blood flow can be measured.

Conventional optical coherence tomography (OCT) systems, such as Doppler OCT can measure the axial component of the velocity while the transversal component is measured by projecting the flow to the optical axis, rather than by direct measurement. Other techniques based on projections involve several assumptions and approximations that limit the utility of those techniques. Notably, the blood flow oriented transversely to the optical axis cannot be measured with acceptable precision. Further, conventional techniques are unable to detect both a direction and a speed of blood flow, including both lateral and axial components of speed. US 2006/132790 A1 discloses an optical coherence tomography system with 3D coherence scanning using at least three optical fibres for illumination and collection. The system creates a 3D fringe pattern scanned dynamically over the object by remote phase shift delays and light modulation to determine the velocity of particles in a medium. Given the complexity of biological tissues, conventional techniques struggle to produce reliable results. In cases where the blood speed needs to be monitored accurately and particularly where accurate measurement of flow in the transverse orientation is required, such as in a surgery, such limitations impair the overall efficiency of the process.

Accordingly, there is a need for improvement.

### SUMMARY

In accordance with one aspect, there is provided a method for determining a velocity of one or more objects in a medium, the method comprising inputting a wave into a wave interference network, generating a first point spread function (PSF) and at least one second PSF distinct from the first PSF, outputting, via the wave interference network, at least one propagation mode of the wave to the medium for illuminating the medium therewith, collecting, via the wave interference network, a scattered signal from the medium, acquiring a first signal having the first PSF associated therewith and at least one second signal having the at least one second PSF associated therewith, determining a first correlation of at least one of the first signal and the at least one second signal, and a second correlation of at least one of the first signal and the at least one second signal, determining a ratio between the first correlation and the second correlation, and determining the velocity of the one or more objects in the medium based on the ratio.

In some embodiments, the method further comprises determining, based on the ratio, a direction of a flow of the one or more objects in the medium.

In some embodiments, generating the first PSF and the at least one second PSF comprises separating, via the wave interference network, at least one first propagation mode and at least one second propagation mode of the wave, the first PSF characterized by the first step fiber propagation mode and the at least one second PSF characterized by the at least one second step fiber propagation mode.

In some embodiments, the first propagation mode and the at least one second propagation mode are separated using a modally specific photonic lantern (MSPL) provided in the wave interference network to separate a fundamental linearly-polarized (LP) LP01 mode from a LP11 mode into two separate fibers of the wave interference network.

In some embodiments, the method further comprises receiving at least one reference signal generated by at least one reference mirror upon reflecting the wave, and generating, via the wave interference network, at least one interference pattern between the scattered signal and the at least one reference signal for acquiring the first signal and the at least one second signal based on the at least one interference pattern.

In some embodiments, the wave is received from a source via the wave interference network comprising a few-mode fiber network provided as part of a Few-Mode Optical Coherence Tomography (FM-OCT) imaging setup.

In some embodiments, the wave is received from a source via the wave interference network comprising a few-mode fiber network provided as part of a laser speckle imaging setup.

In some embodiments, the scattered signal is one of backscattered and forward scattered by the medium.

In some embodiments, the first correlation and the second correlation are determined for a same time delay.

In accordance with another aspect, there is provided a system for determining a velocity of one or more objects in a medium, the system comprising a light source configured to emit a wave excitation, a wave interference network coupled to the light source and configured to receive the wave excitation, generate a first point spread function (PSF) and at least one second PSF distinct from the first PSF, and output at least one propagation mode of the wave to the medium for illuminating the medium therewith; and a computing device coupled to the wave interference network and configured to collect a scattered signal from the medium, acquire a first signal having the first PSF associated therewith and at least one second signal having the at least one second PSF associated therewith, determine a first correlation of at least one of the first signal and the at least one second signal, and a second correlation of at least one of the first signal and the at least one second signal, determine a ratio between the first correlation and the second correlation, and determine the velocity of the one or more objects in the medium based on the ratio.

In some embodiments, the computing device is further configured to determine, based on the ratio, a direction of a flow of the one or more objects in the medium.

In some embodiments, the computing device is configured to generate the first PSF and the at least one second PSF by separating, via the wave interference network, at least one first propagation mode and at least one second propagation mode of the wave, the first PSF characterized by the first step fiber propagation mode and the at least one second PSF characterized by the at least one second step fiber propagation mode.

In some embodiments, the computing device is configured to determine the first correlation and the second correlation for a same time delay.

In some embodiments, the wave interference network comprises a modally specific photonic lantern (MSPL) configured to separate a fundamental linearly-polarized (LP) LP01 mode from a LP11 mode into two separate fibers of the wave interference network.

In some embodiments, the system further comprises at least one reference mirror configured to reflect the wave for generating at least one reference signal, further wherein the computing device is configured to generate, via the wave interference network, at least one interference pattern between the scattered signal and the at least one reference signal for acquiring the first signal and the at least one second signal based on the at least one interference pattern.

In some embodiments, the wave interference network comprises a few-mode fiber network provided as part of a Few-Mode Optical Coherence Tomography (FM-OCT) imaging setup.

In some embodiments, the wave interference network comprises a few-mode fiber network provided as part of a laser speckle imaging setup.

In some embodiments, the light source comprises at least one single-mode port for emitting into the wave interference network the wave excitation comprising single-mode light.

In some embodiments, the light source comprises at least one multimode port for emitting into the wave interference network the wave excitation comprising multimode light.

In some embodiments, the system further comprises a plurality of detectors configured to receive the scattered signal from the medium and to transmit the scattered signal to the computing device.

Many further features and combinations thereof concerning embodiments described herein will appear to those skilled in the art following a reading of the instant disclosure.

### DESCRIPTION OF THE FIGURES

In the figures,
Fig. 1 is a schematic diagram illustrating a sensing system, in accordance with an illustrative embodiment;
Fig. 2A is a schematic diagram illustrating the sample of Fig. 1 illuminated by the source of Fig. 1, in accordance with an illustrative embodiment;
Fig. 2B is a schematic diagram illustrating the sample of Fig. 1 illuminated by the source of Fig. 1, in accordance with another illustrative embodiment;
Fig. 3 is a flowchart illustrating an example method for velocity measurement, in accordance with an illustrative embodiment;
Fig. 4A is a plot illustrating correlation functions generated using the method of Fig. 3, in accordance with an illustrative embodiment;
Fig. 4B is a plot illustrating a velocity profile for a milk sample, as obtained using the method of Fig. 3, in accordance with an illustrative embodiment;
Fig. 4C is a plot comparing results obtained using the method of Fig. 3 to results obtained using existing techniques, in accordance with an illustrative embodiment; Fig. 5 is a plot illustrating ratio functions generated using the method of Fig. 3, in accordance with an illustrative embodiment; and
Fig. 6 is a block diagram of an example computing device, in accordance with an illustrative embodiment.

### DETAILED DESCRIPTION

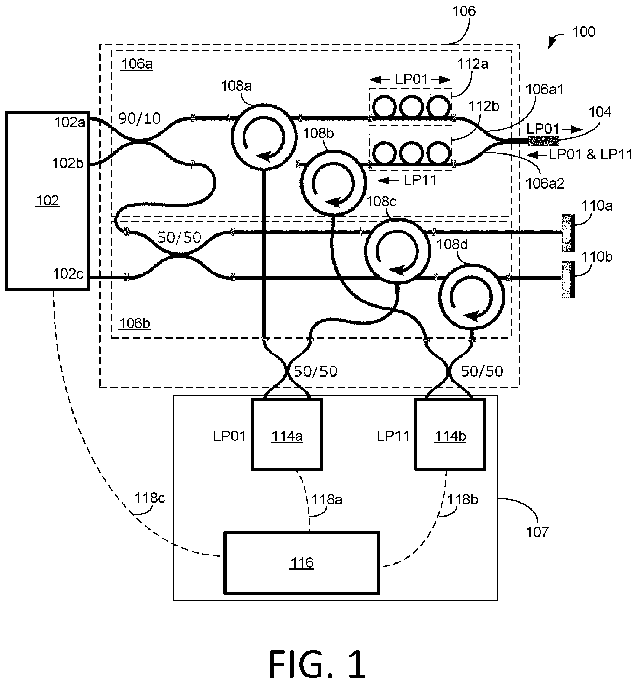

Fig. 1 shows a sensing system 100 used for velocity measurement, according to an illustrative embodiment. As will be described further below, the sensing system 100 is used to implement a sensing technique for velocity measurement. In one embodiment, the sensing system 100 uses few-modes optical-coherence tomography (FM-OCT) to calculate a ratio of correlations between two signals with distinct point spread functions (PSFs). It should however be understood that other interferometric sensing technologies that use signal correlation to determine velocity, including, but not limited to, laser flowmetry, phase microscopy, super resolution microscopy, OCT, laser velocimetry, radar, and LIDAR, may apply. It will be appreciated that the embodiments presented herein use optical components and operate using optical waves. However, the present technology is not bound to the optical spectrum, as any electromagnetic waves, e.g. radio waves, may apply.

The sensing system 100 may comprise any suitable wave interference network used to create signals with distinct PSFs. In some embodiments, the sensing system 100 may comprise a free space spatial mode multiplexer. In other embodiments, a variable focused beam expander may be used before an imaging lens to create two distinct PSFs. In yet other embodiments, the sensing system 100 may comprise a fiber network. In the illustrated embodiment, the sensing system 100 comprises a light source 102 for illuminating a sample 104 via a few-mode (FM) fiber network 106, reference mirrors 110a and 110b, and a detection and analysis system 107. Although reference is made herein to the sensing system 100 comprising a FM fiber network 106 for implementing FM-OCT, it should however be understood that any other suitable wave interference network or system may apply and any suitable wave excitation may be used. For example, bright and dark field optical coherence tomography (BRAD-OCT) may apply. In another embodiment, laser speckle imaging may be used. In yet other embodiments, a silicon-based waveguide network, free-space (as discussed above), or a combination of both could be used.

In some embodiments, the sensing system 100 may comprise at least one modally specific photonic lantern (MSPL) that is used to create distinct PSFs by separating the propagation modes, also referred to herein as "step fiber propagation modes", (e.g., the first two modes, namely the linearly-polarized modes LP01 and LP11) of the FM fiber into distinct fibers for interference, as will be described further below. It will be appreciated that, as used herein, the term "mode" refers to one of the possible orthogonal electromagnetic field configurations that are guided in the step fiber. Any wavefront may be described as a unique combination of those modes regardless of whether it is in the step fiber, another type of fiber or in free space. In the illustrated embodiment, the sensing system 100 comprises two OCT systems which are used to analyze the separated signal, resulting in combined OCT systems with two distinct PSFs. A ratio of correlations is then computed to determine a velocity measurement, as will also be described further below. In some embodiments, the ratio of correlations is a ratio between an autocorrelation of a first signal and a cross-correlation of the first signal and a second signal. Although reference is made herein to the MSPL being used to create two distinct PSFs by separating the first two propagation modes of the FM fiber, it should be understood that more than two propagation modes (e.g., the first three modes) may be used. In addition, any combination of propagation modes (e.g., second and third modes) may be used for illumination and detection. Also, more than one propagation mode may be used for illumination and a single propagation mode may be used for detection. In addition, any suitable technique or device (other than the MSPL) may be used to separate the propagation modes to create distinct PSFs, as discussed previously.

A photonic lantern is understood to be a fiber coupler that adiabatically merges several single-mode waveguides into one multimode waveguide. In other words, the photonic lantern is an N-by-one fiber optic component that maps the propagation modes of a bundle of N single-mode fibers (SMFs) to the modes of a multimode structure. The modally specific photonic lantern is a variant of the photonic lantern that has little or no crosstalk and is ideal for mode control. It provides a low-loss interface between single-mode and multimode for a large bandwidth (e.g., ≥ 100 nm) and allows parallel measurement and control on mode propagation. One example embodiment of such a photonic lantern is described in International Patent Application Publication No. WO 2021/151194 A1, the entire contents of which are incorporated herein by reference. In some embodiments, the photonic lantern is implemented using the embodiments described in International Patent Application Publication No. WO 2019/148276, the entire contents of which are incorporated herein by reference. A MSPL is a sub-category of photonic lanterns that features a one-to-one mapping between individual SMFs and LP modes of a multimode fiber. The modal mapping does not depend on the excitation wavelength, making MSPLs wavelength independent.

The light source 102 may comprise one or more single-mode input/output ports, for instance single-mode input/output ports 102a and 102b, for emitting single-mode light. In some embodiments, the light source 102 may comprise at least one multimode input/output port, for instance multimode input/output port 102c, for emitting multimode light. The light source 102 may be configured to emit single-mode and/or multimode light into the FM fiber network 106. While two (2) single-mode input/output ports 102a and 102b and one (1) multimode input/output port 102c are shown in Fig. 1, this is for illustrative purposes only and the light source 102 may comprise any suitable number of single-mode input/output ports and/or multimode input/output ports, depending on the application. In some embodiments, the sensing system 100 may additionally be configured to collect single-mode and/or multimode light (e.g., backscattered from the sample 104). For instance, the sensing system 100 may be configured to collect backscattered single-mode light via single-mode input/output ports 102a and 102b, and/or to collect backscattered multimode light at multimode input/output port 102c.

In some embodiments, the FM fiber network 106 may comprise multi-clad optical fiber with a taper portion, as described in U.S. Patent No. 11,280,965, the entire contents of which are incorporated herein by reference. The FM fiber network 106 may comprise a combination of single-mode (SM) and multimode (MM) fiber, depending on the application. The FM fiber network 106 illustratively comprises a first set of fibers (also referred to herein as a "sample arm") 106a and a second set of fibers (also referred to herein as a "reference arm") 106b. Via the sample arm 106a, the FM fiber network 106 transmits single-mode and/or multimode light from the light source 102 to the sample 104 and transmits backscattered light from the sample 104 to the detection and analysis system 107. Via the reference arm 106b, the FM fiber network 106 transmits single-mode and/or multimode light from the light source 102 to the reference mirrors 110a and 110b and transmits reflected light from the reference mirrors 110a and 110b to the detection and analysis system 107.

A plurality of optical circulators, for instance optical circulators 108a, 108b, 108c, and 108d, may be provided within the FM fiber network 106. In some embodiments, the optical circulators 108a and 108b may be used to transmit backscattered light from the sample 104, via the sample arm 106a, to the detection and analysis system 108, for instance to detectors 114a and 114b. In some embodiments, the optical circulators 108c and 108d may be used to transmit reflected light from the reference mirrors 110a and 110b, via the reference arm 106b, to the detection and analysis system 107, for instance to detectors 114a and 114b. In the embodiment of Fig. 1, the optical circulators 108a, 108b, 108c, and 108d are three-port optical devices, although it should be understood that four-port devices may also apply. In some embodiments, the optical circulators 108a, 108b, 108c, and 108d are fiber-optic circulators enabling bi-directional transmission. In some embodiments, the optical circulators 108a, 108b, 108c, and 108d are polarization-maintaining fiber optical circulators.

A plurality of polarization controllers, for instance polarization controllers 112a and 112b, may be provided within the FM fiber network 106 for controlling and/or modifying the polarization state of the light via the FM fiber network 106. In some embodiments, the polarization controllers 112a and 112b are provided on the sample arm 106a for controlling and/or modifying the polarization state of the transmitted light incident upon and backscattered from the sample 104. In some embodiments, the polarization controllers 112a and 112b are fiber polarization controllers. In some alternative embodiments (e.g., where devices other than an FM-OCT are used), the sensing system 100 does not comprise polarization controllers 112a, 112b.

The detection and analysis system 107 may comprise a plurality of detectors, for instance detectors 114a and 114b, and a computing device 116 communicatively coupled to the detectors 114a and 114b and to the light source 102 via communication links 118a, 118b, and 118c, respectively. In some embodiments, the detection and analysis system 107 may be communicatively coupled to other components of the sensing system 100, for instance to the polarization controllers 112a and 112b, and/or to the optical circulators 108a, 108b, 108c, and 108d. In some embodiments, the computing device 116 may send control instructions to components of the sensing system 100, for instance to the detectors 114a and 114b via the communication links 118a and 118b, respectively, and/or to the light source 102 via the communication link 118c.

In some embodiments, the detectors 114a and 114b comprise photodetectors and/or spectrometers. The detectors 114a and 114b may receive backscattered light from the sample 104 via optical circulators 108a and 108b provided in the sample arm 106a of the FM fiber network 106. The detectors 114a and 114b may also receive, via the optical circulators 108c and 108d provided in the reference arm 106b of the FM fiber network 106, light reflected by the reference mirrors 110a and 110b. In some embodiments, the detectors 114a and 114b may carry out processing or post-processing of the received light. In some embodiments, the detectors 114a and 114b may implement analog-to-digital (ADC or A/D) conversion of the received light prior to transmission to the computing device 116. In some embodiments, the detectors 114a and 114b may be partially or wholly integrated into the computing device 116 of the detection and analysis system 107. In some embodiments, the detectors 114a and 114b and/or the computing device 116 may be configured to calculate a correlation, a cross-correlation and/or autocorrelation of the received light, as described in further detail herein below.

Communication between the detectors 114a and 114b and the computing device 116, and between the computing device 116 and the light source 102, may occur across wired, wireless, or a combination of wired and wireless networks. The networks may be any type of network or combination of networks for carrying data communications. Such a network may comprise, for example, a Personal Area Network (PAN), Local Area Network (LAN), Wireless Local Area Network (WLAN), Metropolitan Area Network (MAN), or Wide Area Network (WAN), such as the Internet, or combinations thereof. In the embodiment of Fig. 1, communication occurs across the communication links 118a, 118b, and 118c. In some embodiments, the communication links 118a, 118b, and 118c may comprise one or more communications cables, for instance coaxial cable, twisted pair cable, or fiber optic cable, among other possibilities.

In some embodiments, the sample 104 may comprise biological tissue, provided in either *in vivo* or ex *vivo* conditions. In some embodiments, the sample 104 may comprise a retina of an eye, a brain sample, or a lung sample. The sample 104 may have a flow associated therewith. For example, the sample 104 may comprise blood flowing through an artery or blood vessel. The sample 104 may comprise organic material, for example milk, flowing through a micro-channel. The sample 104 may also comprise inorganic material. For example, the sample 104 may comprise humans in a crowd, cars in traffic, etc. It should therefore be understood that, as used herein, the term "sample" (as in the sample 104), which is used interchangeably with the term "medium", refers to any suitable substance composed of a plurality of scatterers in a medium, or a plurality of individual objects within a moving environment. In some embodiments, the flow may be characterized by a diffusion coefficient, as described in further detail herein below. Multiple different sources that can cause flow may apply and the flow may therefore be characterized by any suitable parameter other than diffusion.

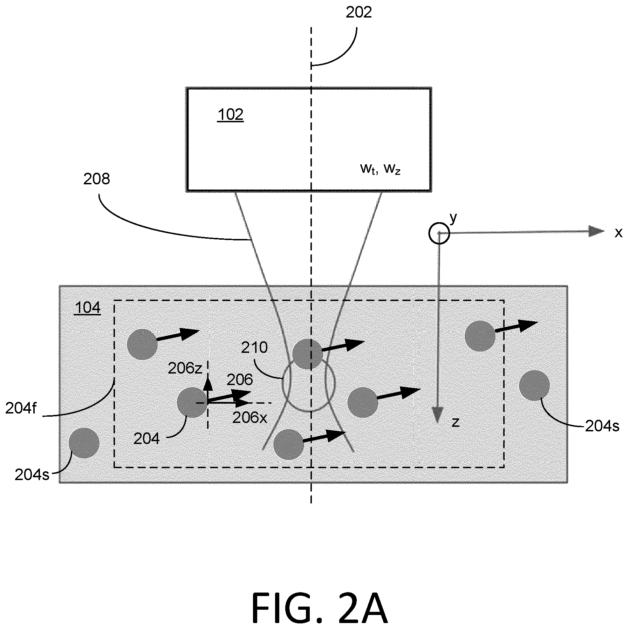

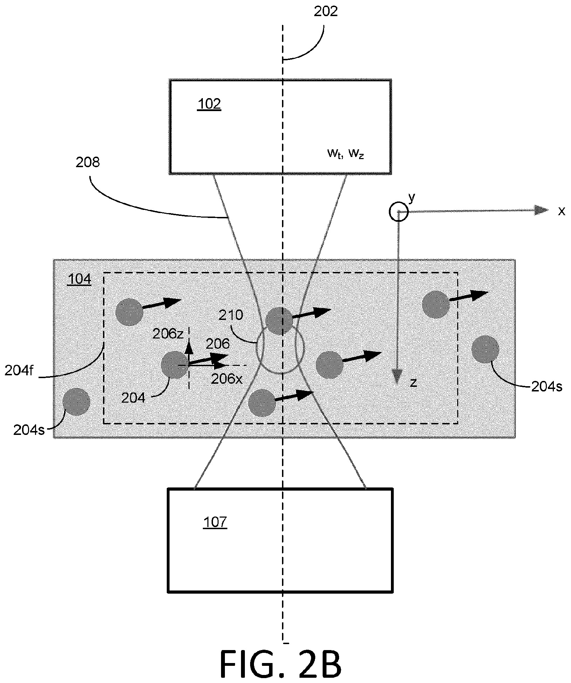

Figs. 2A and 2B show a sample, for instance the sample 104, being illuminated by a light source, for instance the light source 102 along an optical axis 202. The FM fiber network (reference 106 in Fig. 1) is not shown for sake of clarity. The sensing system 100 may be characterized by a transverse resolution *wₜ* and an axial resolution *w_{z}*, one or both of which may be adjustable depending on the equipment used. While, in one embodiment, the axial resolution *w_{z}* is a product of the sensing system 100 and the light source 102, in the case of swept source OCT (as illustrated in Fig. 1 where FM-OCT is used), the axial resolution *w_{z}* may be mainly defined by the light source 102. An (x, y, z) coordinate system may be defined, where the z direction (also referred to herein as the "axial" direction) is directed parallel to the optical axis 202, and the x and y directions (also referred to herein as the "lateral" and "transverse" directions, respectively) are orthogonal to the optical axis 202. The sample 104 may comprise a plurality of particles 204 having a velocity represented by velocity vector 206. The velocity vector 206 may have both an axial component 206z, a lateral component 206x, and a transverse component (not shown). In other words, the velocity vector 206 may be three-dimensional, with components in the x, y, and z directions. The magnitude of the velocity vector 206, or speed, may be calculated knowing its components in the x, y, and z directions. At least some of the particles 204 may be in movement, thus forming a flow 204f circulating through the sample 104. At least some of the particles 204, for instance the particles 204s, may be static (i.e. not moving, and thus not forming the flow 204f). The particles 204 may also have varying directions. Varying direction will result in the average velocity direction of the different velocities. However, if two particles as in 204 are moving in the axial direction at different speeds, in the same pixel resolution, and at the same time, this may cause another source of flow measurement error, which the technique proposed herein is robust against.

As shown in Fig. 2A, the light source 102 may illuminate the sample 104 with a light beam 208 and the sensing system 100 may then collect light backscattered from the sample 104. In the illustrated embodiment, the beam 208 is concentrated in the shape of a PSF 210 projected onto the sample 104. The PSF 210 may correspond to a scaled version of the propagation mode of the light emitted by the light source 102 incident upon the sample 104. In other words, a different propagation mode may have a different PSF. For example, if the sample 104 is illuminated with the LP fundamental mode LP01, the PSF 210 may be substantially circular (as illustrated in Fig. 2). If the sample 104 is illuminated with the LP mode LP11, the PSF 210 may comprise two separate lobes. In other words, the LP11 mode comprises two lobes while the LP01 mode comprises only one lobe. As particles 204 circulate through the sample 104, they may intersect the PSF 210, thus altering the characteristics of the backscattered light, as further described herein below. It will be appreciated that light travelling across the two lobes of the LP11 mode goes from having a positive response, to zero, to a negative response.

While the embodiment presented in Fig. 2A shows a system that projects light from the light source 102 to the sample 104 and collects backscattered light from the sample 104, other embodiments may apply. For instance, as presented in Fig. 2B, the light source 102 may be positioned on one side of the sample 104, and forward scattered light is collected and analysed by the detection and analysis system 107. It will be thus understood that the present technology is not bound to light backscattered from the sample 104.

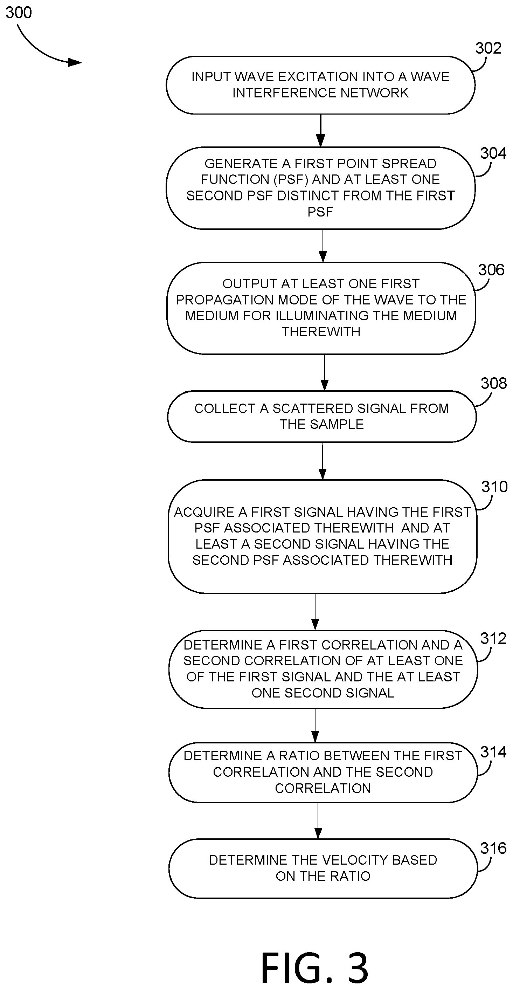

Referring now to Fig. 3, with additional reference to Figs. 1, 2A and 2B, a method 300 for velocity measurement will be described in accordance with one embodiment. The method 300 may be performed at the detection and analysis system (reference 107 in Fig. 1), and more specifically at the computing device (reference 116 in Fig. 1). At step 302, a wave excitation (e.g. light from a light source, such as the light source 102 in Fig. 1) is input into a wave interference network (e.g., FM fiber network 106 of Fig. 1). At step 304, a first PSF and at least one second PSF distinct from the first PSF are generated. This may be achieved by separating a first propagation mode and at least one second propagation mode of the wave within the wave interference network. In other embodiments, the first propagation mode and the at least one second propagation mode of the wave are separated digitally. As described herein above, this may be achieved using the MSPL provided within the FM fiber network 106 (provided as part of an FM-OCT imaging setup for instance), the MSPL separating the two modes into two distinct fibers for interference. In some embodiments, the first mode comprises the LP01 mode (characterizing the first PSF), and the second mode comprises the LP11 mode (characterizing the second PSF). In some embodiments, at least one additional mode may be generated, for instance LP21. Other possibilities may apply, for instance wherein different modes than LP01, LP11, and LP21 are generated. For example, when BRAD-OCT is used, the LP01 mode may be used to illuminate the sample and scattered light may be collected from LP01, LP11, and LP21.

At step 306, at least one of first propagation mode (and optionally at least one second propagation mode) is output to a medium (e.g. the sample 104 of Fig. 1) for illuminating the medium therewith. In some embodiments, in order to achieve two PSFs, only the first propagation mode is output to the medium for illumination thereof and collection of the scattered signal (step 308) is done with two propagation modes (i.e. the medium generates a combination of propagation modes two of which are collected and detected, e.g., by FM-OCT). As discussed above with reference to Fig. 1, in one embodiment, the first propagation mode can be transmitted to the sample 104 via the sample arm 106a of the FM fiber network 106. In another embodiment, illumination may be done using two propagation modes and collection using a single propagation mode. In yet another embodiment, illumination may be done using two propagation modes and collection using two propagation modes.

Step 308 comprises collecting, via the wave interference network, a scattered signal from the sample. In one embodiment, the scattered signal is collected from at least one of the first propagation mode and the at least one second propagation mode. It will be appreciated that scatterers in the sample may induce interference in the scattered signal. As illustrated in Fig. 1, in one embodiment, the scattered signal (i.e. light) is split into a first component collected by a first fiber 106a1 of the sample arm 106a, and a second component collected by a second fiber 106a2 of the sample arm 106a. In some embodiments, the first component may pass through the polarization controller 112a (in cases where such a polarization controller 112a is provided) and the optical circulator 108a and be transmitted to the detector 114a of the detection and analysis system 107, and the second component may pass through the polarization controller 112b and the optical circulator 108b and be transmitted to the detector 114b of the detection and analysis system 107.

Step 310 comprises acquiring a first signal having the first PSF associated therewith and at least a second signal having the second PSF associated therewith. This may be achieved by generating, via the wave interference network, at least one interference pattern between the scattered signal and at least one reference signal. In one embodiment, a first image and at least a second image are acquired simultaneously in response to the at least one interference pattern being generated. In one embodiment, the at least one reference signal may be generated from light reflected by at least one reference mirror, as described herein above with reference to Fig. 1. The at least one reference signal can be collected via the reference arm 106b of the FM fiber network 106 and subsequently transmitted to the detection and analysis system 107. It should however be understood that, in some embodiments that do not involve OCT imaging, no reference signal may be used.

At step 312, a first correlation (which may be referred to herein as an "autocorrelation") of at least one of the first signal collected from the first propagation mode and the at least one second signal (e.g., a correlation of the first signal with itself) is determined, and a second correlation (which may be referred to herein as a "cross-correlation") between at least one of the first signal and the at least one second signal collected from the at least one second propagation mode is determined. The autocorrelation and the cross-correlation are determined for a same time delay *τ*. At step 314, a ratio between the autocorrelation and the cross-correlation is determined. At step 316, the velocity is determined based on the ratio determined at step 314.

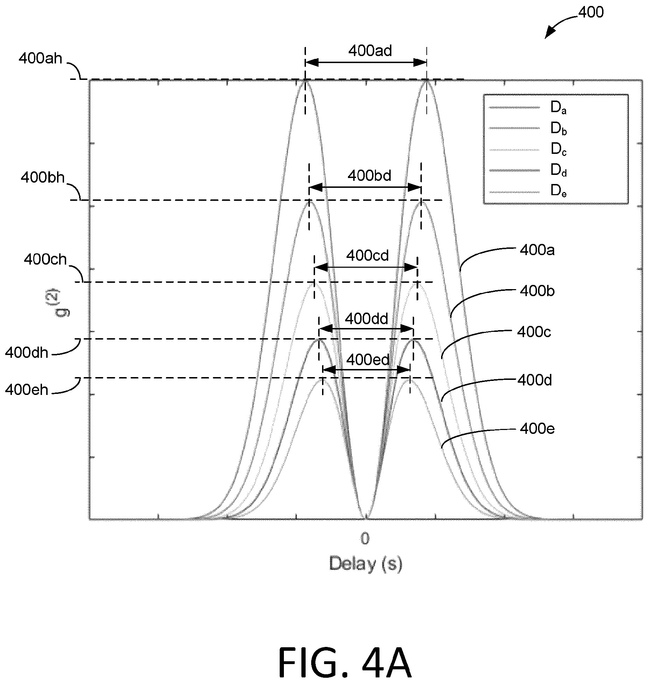

Fig. 4A shows plots generated using the method 300, wherein the first mode is LP01 and the second mode is LP11. Fig. 4A illustrates correlation functions 400a, 400b, 400c, 400d, and 400e of signals detected by LP01 and LP11, as a function of a time delay *τ* for different diffusion coefficients Da, Db, Dc, Dd, and De, respectively, where Da < Db < Dc < Dd < De. The diffusion coefficients Da, Db, Dc, Dd, and De may be expressed using any convenient units, for instance um²/s, where um stands for micrometers. The y-axis may be representative of the degree of correlation between different peaks, i.e., that a Y value of one (1) corresponds to a perfect correlation between different peaks, and a Y value of zero (0) corresponds to a prefect decorrelation (i.e. no correlation) between different peaks. The correlation functions 400a, 400b, 400c, 400d, and 400e may differ in shape and/or magnitude when modes other than LP01 and LP11 are used, or for non-FM-OCT embodiments of the sensing system 100.

In a conventional OCT system where only a first component corresponding to LP01 is collected, the first order correlation (or autocorrelation) *g*⁽¹⁾(*τ*) of the signal as a function of a time delay *τ* may be calculated as follows: ${g}^{\left(1\right)} \left(τ\right) = {M}_{S} + {M}_{F} {e}^{- \left({ν}_{1}^{2}{τ}^{2}\right) / {u}_{1} - \left({ν}_{z}^{2}{τ}^{2}\right) / wz} {e}^{- {\left(2{k}_{0}\right)}^{2} {Dτ}_{e} i 2 {kν}_{z}}$ (1)
where *M_{S}* is the proportion of static particles, for instance the particles 204s, *M_{F}* is the proportion of particles involved in the flow, for instance the plurality of particles 204 in the flow 204f, *vₜ* is the velocity in the transverse direction, *v_{z}* is the velocity in the z direction (i.e. axial speed), *wₜ* is the beam waist in the transverse direction, *w_{z}* is the resolution in the z direction, *k*₀ is the central wave number, and *D* is the diffusion coefficient.

The diffusion coefficient *D* may be difficult to determine, as it depends on multiple factors.

It is possible to re-write Equation (1) in the following form: ${g}^{\left(1\right)} \left(τ\right) = \frac{\left〈\left|S\right|\right〉}{\left〈\left|S+N\right|\right〉} \left[{M}_{S}+\left(1-{M}_{S}\right){g}_{{f}_{xy}}\left(τ\right){g}_{{f}_{z}}^{\left(1\right)}\left(τ\right){g}_{D}^{\left(1\right)}\left(τ\right)\right]$ (2)
where $\frac{\left〈\left|S\right|\right〉}{\left〈\left|S+N\right|\right〉}$ is a ratio of the average value of the signal to the average value of the signal-plus-noise (i.e. $\frac{\left〈\left|S\right|\right〉}{\left〈\left|S+N\right|\right〉}$ is indicative of which proportion of the noisy signal that is measured is not noise) , ${g}_{D}^{\left(1\right)}$ is the decorrelation due to diffusion, ${g}_{{f}_{z}}^{\left(1\right)}$ is the decorrelation due to axial speed, and ${g}_{{f}_{xy}}^{\left(1\right)}$ is the decorrelation due to lateral speed. $\frac{\left〈\left|S\right|\right〉}{\left〈\left|S+N\right|\right〉}$may be estimated a *priori,* using any suitable technique, and *M_{S}* (the proportion of static particles) can be determined by computing the autocorrelation by LP01 for a large time delay.

By considering the heights 400ah, 400bh, 400ch, 400dh, and 400eh of the peaks and the time delays between peaks 400ad, 400bd, 400cd, 400dd, and 400ed of the correlation functions 400a, 400b, 400c, 400d, and 400e, respectively, the value of the diffusion coefficients Da, Db, Dc, Dd, and De may be calculated. More generally, the value of any diffusion coefficient D may be determined based on the peak height and on the time delay between peaks. The time delay between peaks represents the time taken by a particle to travel from one lobe of the LP11 mode to the other lobe. The distance between lobes being known, the ratio of the distance between lobes to the time delay between peaks may then be computed to measure the velocity.

It is also proposed herein to use ratios of correlations to determine velocity, as described herein above. If ${g}_{11}^{\left(1\right)}$ is defined as the autocorrelation of a first signal collected from the first PSF to itself after a delay of *τ*, and ${g}_{12}^{\left(1\right)}$ is defined as the cross-correlation of the first signal collected from the first PSF and a second signal collected from the second PSF, the ratio of the two correlations may be expressed as follows: ${g}_{11}^{\left(1\right)} \left(τ\right) / {g}_{12}^{\left(1\right)} \left(τ\right) = \frac{{g}_{11}^{\left(1\right)} {f}_{xy}}{{g}_{12}^{\left(1\right)} {f}_{xy}}$ (3)
since all other terms in Equation (2) cancel out except ${g}_{11 {f}_{xy}}^{\left(1\right)}$ and ${g}_{12 {f}_{xy}}^{\left(1\right)}$, which are dependent on the lateral speed 206x and on the topology of the sensing system 100. The topology of the sensing system 100 being known, the lateral speed 206x may be calculated.

Similarly, starting from Equation (1), by adjusting the resolution *w_{z}* in the z direction (for instance, by reducing the bandwidth of the light source 102) to obtain a reduced beam waist *w_{rz}* the ratio of ${g}_{{sf}_{z}}^{\left(1\right)}$, the decorrelation of the backscattered light due to an axial speed (z axis) for an axial resolution *w_{z}*, to ${g}_{{f}_{z}}^{\left(1\right)}$, the decorrelation of the backscattered light due to the axial speed (z axis), may be calculated as follows: $\frac{{g}_{{}_{{sf}_{z}}}^{\left(1\right)}}{{g}_{{}_{{f}_{z}}}^{\left(1\right)}} = {c}^{- \left({v}_{z}^{2}{τ}^{2}\right) \left(\frac{1}{{w}_{rz}^{2}}-\frac{1}{{w}_{z}^{2}}\right)}$ (4)

Equation (4) is uniquely dependent on velocity and on the known resolution of the sensing system 100.

Equation (2) above may be rewritten as follows: ${g}^{\left(1\right)} \left(τ\right) = exp \left[-i2{nk}_{0}{ν}_{z}τ\right] ⋅ exp \left[-4{n}^{2}{k}_{0}^{2}{D}_{Τ}τ\right] ⋅ exp \left[-\frac{{ν}_{x}^{2} {τ}^{2}}{{w}_{xy}^{2}}\right] ⋅ exp \left[-\frac{{ν}_{y}^{2} {τ}^{2}}{{w}_{xy}^{2}}\right] ⋅ exp \left[-\frac{{ν}_{z}^{2} {τ}^{2}}{{w}_{z}^{2}}\right]$ (5)
where n is the refractive index, *vₓ* is the velocity in the x direction, *v_{y}* is the velocity in the y direction, *w_{xy}* is the beam waist in the x or y direction.

Equation (5) can be simplified into separate correlation terms, as follows: ${g}^{\left(1\right)} \left(τ\right) = {g}_{D}^{\left(1\right)} \left(τ\right) ⋅ {g}_{x}^{\left(1\right)} \left(τ\right) ⋅ {g}_{y}^{\left(1\right)} \left(τ\right) ⋅ {g}_{z}^{\left(1\right)} \left(τ\right)$ (6)

There are other effects to be considered that affect velocity, as follows: ${g}^{\left(1\right)} \left(τ\right) = {g}_{D}^{\left(1\right)} \left(τ\right) ⋅ {g}_{{v}_{x}}^{\left(1\right)} \left(τ\right) ⋅ {g}_{{v}_{y}}^{\left(1\right)} \left(τ\right) ⋅ {g}_{{v}_{z}}^{\left(1\right)} \left(τ\right) ⋅ {g}_{\partial z}^{\left(1\right)} ⋅ {g}_{T}^{\left(1\right)} \left(τ\right) ⋅ {g}_{S}^{\left(1\right)} \left(τ\right)$ (7)

It can be seen that, when the ratio of correlations is computed, several factors from equation (7) cancel out and only the factors ${g}_{{v}_{x}}^{\left(1\right)} \left(τ\right) , {g}_{{v}_{y}}^{\left(1\right)} \left(τ\right)$, and ${g}_{{\partial }_{z}}^{\left(1\right)} \left(τ\right)$ remain, with the factors ${g}_{{\partial }_{z}}^{\left(1\right)} \left(τ\right)$ and ${g}_{S}^{\left(1\right)} \left(τ\right)$ being attenuated and approximated to one (1). The factor ${g}_{{\partial }_{z}}^{\left(1\right)} \left(τ\right)$ results from (i.e. is representative of) scatterers moving at different axial speeds in the same voxel. The factor ${g}_{S}^{\left(1\right)} \left(τ\right)$ is representative of a so-called "shadow artefact" where the random motion of scatterers causes a decorrelation of the signal in the pixels below their own location. The factor ${g}_{T}^{\left(1\right)} \left(τ\right)$ results from (i.e. is representative of) tumbling of the scatterers.

The LP modes are then approximated as follows: $\begin{matrix}LP 01 : {h}_{LP 01} = exp \left[-\frac{{x}^{2}}{{w}_{xy}^{2}}\right] \\ LP 11 : {h}_{LP 11} = \frac{r}{0 , 702 {w}_{xy}} exp \left[-\frac{{r}^{2}}{{\left(0,926{w}_{x , y}\right)}^{2}}\right] cos \left(θ\right) \\ = \frac{x}{0 , 702 {w}_{xy}} exp \left[-\frac{{x}^{2} + {y}^{2}}{{\left(0,926{w}_{xy}\right)}^{2}}\right]\end{matrix}$ (8)

Where *h* is the normalized PSF written as: $h = {h}_{xy} {h}_{z}$ (9)

Where *h_{z}* is the PSF in the z direction and *h_{xy}* is the product of the illumination and detection, and are defined as follows: $\begin{matrix}{h}_{xy} = {h}_{LP 01} * {h}_{LP 01} \left(when both illumination and detection are from LP01\right) \\ {h}_{xy} = {h}_{LP 01} * {h}_{LP 11} \left(when illumination is by LP01 and detection by LP11\right)\end{matrix}$ (10) ${h}_{z} = exp \left[-i2{nk}_{0}{v}_{z}τ\right] ⋅ exp \left|-\frac{{z}^{2}}{{w}_{z}^{2}}\right|$ (11)

For uniform motion in the lateral plane, the following correlation for illumination with LP01 and detection with LP11 is obtained: ${g}^{\left(1\right)} \left(τ\right) \left(l.P11\left(0\right)l.P11\left(τ\right)\right) = \left(1-A \frac{{r}_{A}^{2} {τ}^{2}}{{w}_{XY}^{2}}\right) exp \left|-B \frac{\left({v}_{x}^{2}+{v}_{y}^{2}\right) {τ}^{2}}{{w}_{xy}^{2}}\right|$ (12)
where A and B are constants. In one embodiment, A = 2.17 and B = 1.08.

If two different modes of detection are correlated, the following is obtained: ${g}^{\left(1\right)} \left(τ\right) \left〈I.P11\left(0\right)l.P01\left(τ\right)\right〉 = C \frac{{v}_{x} τ}{{w}_{xy}} exp \left|-D\frac{\left({v}_{x}^{2}+{v}_{x}^{2}\right) {τ}^{2}}{{w}_{xy}^{2}}\right|$ (13)
where C and D are constants. In one embodiment, C is about 1.5 and D is close to 1. For example, in one embodiment, C = 1.41 and D = 1.04.

As previously noted, it is proposed herein to calculate ratios of correlations to measure velocity. In one embodiment, the following ratio of correlations can be computed: $\frac{{g}^{\left(1\right)} \left(τ\right) \left〈l.P11\left(0\right)l.P01\left(τ\right)\right〉}{{g}^{\left(1\right)} \left(τ\right) \left〈l.P01\left(0\right)l.P01\left(τ\right)\right〉} = C \frac{{v}_{x} τ}{{w}_{xy}} exp \left|-E\frac{\left({v}_{x}^{2}+{v}_{y}^{2}\right) {τ}^{2}}{{w}_{xy}^{2}}\right|$ (14)
where E is a constant. In one embodiment, E = 0.04.

It should be understood that the values of constants A, B, C, D, and E provided herein are for illustrative purposes only and that, in embodiments where different PSFs are used, different values may apply for A, B, C, D, and E.

In embodiments where more than two propagation modes are considered, the correlation between additional propagation modes in the presence of flow can be calculated as: ${g}_{\left〈Mode1\left(0\right)Mode2\left(τ\right)\right〉}^{\left(1\right)} \left(τ\right) = {\int }_{- inf}^{inf} {\int }_{- - inf}^{inf} {h}_{xy - 1} \left(x, y\right) ⋅ {h}_{xy - 2} \left(x+{ν}_{x},τ,y+{ν}_{y}τ\right) dxdy$ (15)
where Mode1(0) refers to the PSF of a first mode at time 0 being correlated to the second mode Mode2(*τ*) with a time delay of *τ*. h_{xy-1}(x, y) and h_{xy-2}(x+*vₓτ*, y+*v_{y}τ*) refer to the function of the PSF of these two modes, respectively.

The ratio of correlations described herein may further be used to determine the direction of the flow of one or more objects in a medium (e.g., of particles 204 in sample 104), in addition to being used to determine the velocity (i.e., the speed of the flow).

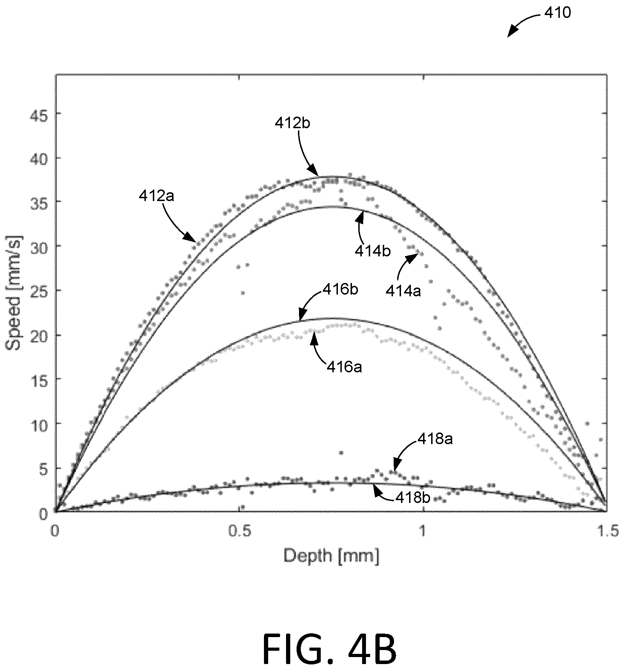

Fig. 4B shows a plot 410 that depicts exemplary velocity profiles (i.e. speed as a function of depth) of particles propagating in a milk sample having a flow associated therewith. In this exemplary embodiment, the particles flow in a tube having an interior diameter of 1.5 mm. The tube is rotated in the transverse plane at a variety of angles and velocity measurements have been obtained (e.g., using the sensing system 100 of Fig. 1) based on a signal backscattered by the milk sample. In plot 410, for each angle, dots (see dotted lines 412a, 414a, 416a, and 418a) represent experimental data (i.e. actual velocity measurements), while solid lines (see lines 412b, 414b, 416b, and 418b) represent expected (i.e. theoretical) velocity profiles. In particular, the results of Fig. 4B represent flow rate measurements taken at angles of -5° (lines 412a, 412b), 25° (lines 414a, 414b), 55° (lines 416a, 416b), and 85° (lines 418a, 418b). From Fig. 4B, it can be seen that the velocity profile measured as a function of depth has a Gaussian shape, with the speed being equal to zero mm/s at a depth of 0 mm and a depth of 1.5 mm, i.e. when particles reach the tube's inner walls.

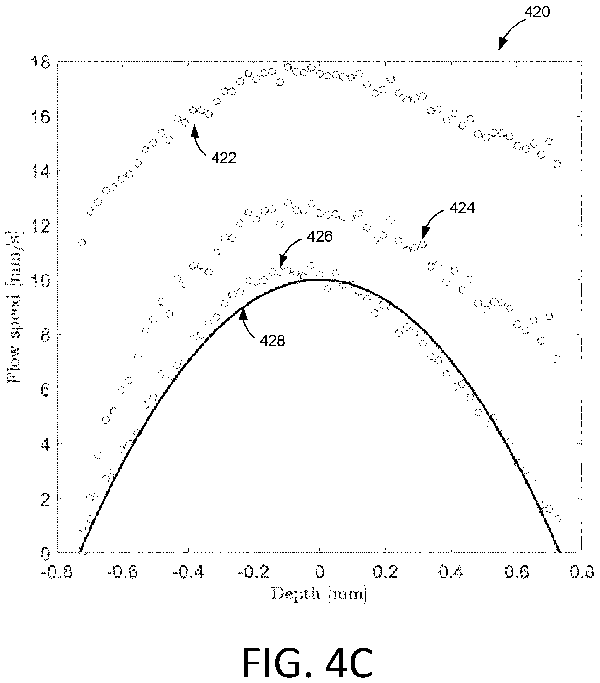

Fig. 4C shows a plot 420 comparing a velocity profile obtained using the method 300 of Fig. 3 with velocity profiles obtained using existing techniques. In the embodiment of Fig. 4C, the velocity profiles are measured for particles propagating in a sample at a flow rate of 0.5 mL/min. In plot 420, dots (see dotted lines 422, 424, 426) represent experimental data (i.e. actual velocity measurements), while the solid line (see line 428) represents an expected (i.e. theoretical) velocity profile. In particular, line 422 represents flow speed measurements obtained using the method 300 of Fig. 3, line 424 represents flow speed measurements obtained using previous technique(s) without diffusion correction, and line 426 represents flow speed measurements obtained using previous technique(s) without diffusion correction. For instance, previous techniques achieve deducing the velocity using only the first mode autocorrelation function as shown in equation (1). In this case, diffusion correction is applied by selecting the value of D in equation (1) in order to match the measured velocity with the expected velocity. It will be appreciated that the results obtained using the methods and systems proposed herein (as shown by dotted line 426) are closer to the theoretical velocity profile (shown by solid line 428) obtained using existing techniques. This may be attributed, *inter alia,* to the fact that the systems and methods proposed herein achieve error cancellation by dividing the autocorrelation factor with the cross-correlation factor.

It will also be appreciated that, generally, a scattered signal obtained from particles that are close to where the light is introduced exhibits lower error (i.e. is closer to the theoretical velocity profile) than a scattered signal obtained from particles that are further away from where the light is introduced. Such behavior may be due to a diminution of the intensity of the signal caused by a low signal.

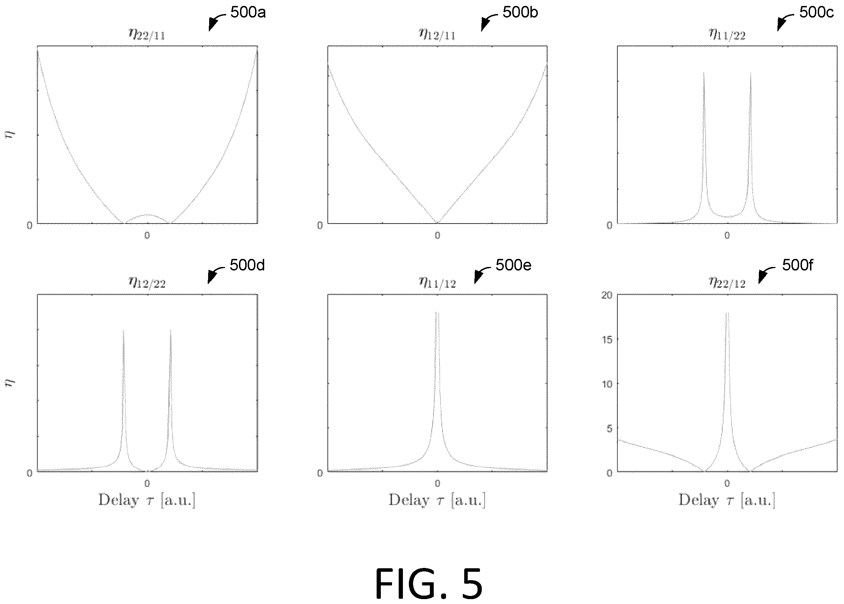

Fig. 5 shows plots 500a, 500b, 500c, 500d, 500e, and 500f generated using the method 300. In particular, Fig. 5 illustrates the possible ratios of correlations obtained with the different cross-correlations and autocorrelations that can be calculated. Each plot 500a, 500b, 500c, 500d, 500e, and 500f thus indicates the ratio of correlations (labelled η in Fig. 5) as a function of the time delay *τ,* where the first propagation mode (labelled with number "1") is LP01 and the second propagation mode (labelled with number "2") is LP11. It can be seen that that diffusion has no impact on the shape of the plots 500a, 500b, 500c, 500d, 500e, and 500f. The ratio of correlations (i.e. η_{12/11}) computed using equation (13) can be seen in plot 500b, which has a slope that depends on the velocity, the PSF width, and the time delay. It should however be understood that any of the ratios of correlations shown in plots 500a, 500b, 500c, 500d, 500e, and 500f (i.e. any one of η_{22/11}, η_{12/11}, η_{11/22}, η_{12/22}, η_{11/12}, or η_{22/12}) may be used to determine flow.

In some embodiments, the ratio of correlation η_{12/11} is used to determine flow. It will be appreciated that the linear or quasi-linear dependency between the ratio of correlation η_{12/11} and the time delay (compared to other ratios of correlation) may allow for a model that is easier to compute, which may in turn prove beneficial.

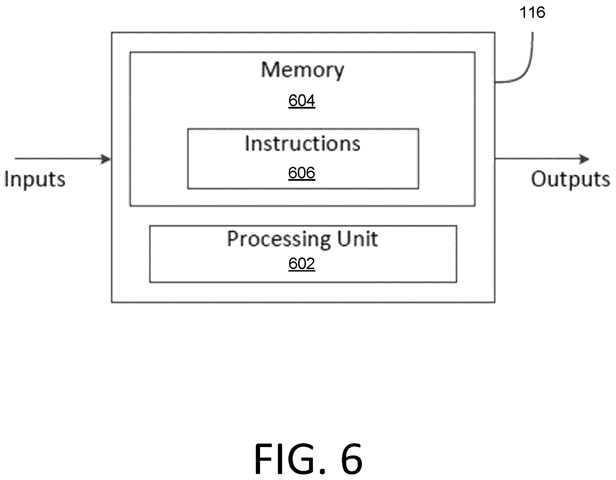

With reference to Fig. 6, part or all of the embodiments of the devices, systems and methods described herein may be implemented in a combination of both hardware and software.

Fig. 6 illustrates an example computing device 116 which may be used to implement the sensing system 100 of Fig. 1 and/or the method 300 of Fig. 3. The computing device 116 comprises a processing unit 602 and a memory 604 which has stored therein computer-executable instructions 606. The processing unit 602 may comprise any suitable devices configured to implement the functionality of the sensing system 100 and/or the method 300 such that instructions 606, when executed by the computing device 116 or other programmable apparatus, may cause the functions/acts/steps performed by the sensing system 100 and/or the method 300 as described herein to be executed. The processing unit 602 may comprise, for example, any type of general-purpose microprocessor or microcontroller, a digital signal processing (DSP) processor, a central processing unit (CPU), an integrated circuit, a field programmable gate array (FPGA), a reconfigurable processor, other suitably programmed or programmable logic circuits, custom-designed analog and/or digital circuits, or any combination thereof.

The memory 604 may comprise any suitable known or other machine-readable storage medium. The memory 604 may comprise non-transitory computer readable storage medium, for example, but not limited to, an electronic, magnetic, optical, electromagnetic, infrared, or semiconductor system, apparatus, or device, or any suitable combination of the foregoing. The memory 604 may include a suitable combination of any type of computer memory that is located either internally or externally to device, for example random-access memory (RAM), read-only memory (ROM), compact disc read-only memory (CDROM), electro-optical memory, magneto-optical memory, erasable programmable read-only memory (EPROM), and electrically-erasable programmable read-only memory (EEPROM), Ferroelectric RAM (FRAM) or the like. Memory 604 may comprise any storage means (e.g., devices) suitable for retrievably storing machine-readable instructions 706 executable by processing unit 602.

The computing device 116 may be any suitable computing device, such as a desktop computer, a laptop computer, a mainframe, a server, a distributed computing system, a portable computing device, a mobile phone, a tablet, or the like. The following discussion provides many example embodiments. Although each embodiment represents a single combination of inventive elements, other examples may include all possible combinations of the disclosed elements. Thus if one embodiment comprises elements A, B, and C, and a second embodiment comprises elements B and D, other remaining combinations of A, B, C, or D, may also be used.

The term "connected" or "coupled to" may include both direct coupling (in which two elements that are coupled to each other contact each other) and indirect coupling (in which at least one additional element is located between the two elements).

As can be understood, the examples described above and illustrated are intended to be exemplary only. The scope is indicated by the appended claims.

## Claims

1. A method (300) for determining a velocity (206) of one or more objects (204) in a medium (104), the method comprising:
inputting a wave into a wave interference network (106);
generating a first point spread function, PSF,
and at least one second PSF distinct from the first PSF;
outputting, via the wave interference network, at least one propagation mode of the wave to the medium for illuminating the medium therewith;
collecting, via the wave interference network, a scattered signal from the medium;
acquiring a first signal having the first PSF associated therewith and at least one second signal having the at least one second PSF associated therewith;
determining a first correlation of at least one of the first signal and the at least one second signal, and a second correlation of at least one of the first signal and the at least one second signal;
determining a ratio between the first correlation and the second correlation; and
determining the velocity of the one or more objects in the medium based on the ratio.

2. The method of claim 1, further comprising determining, based on the ratio, a direction of a flow of the one or more objects in the medium.

3. The method of claim 1 or 2, wherein generating the first PSF and the at least one second PSF comprises separating, via the wave interference network, at least one first propagation mode and at least one second propagation mode of the wave, the first PSF **characterized by** the first step fiber propagation mode and the at least one second PSF **characterized by** the at least one second step fiber propagation mode, preferably wherein the at least one first propagation mode and the at least one second propagation mode are separated using a modally specific photonic lantern, MSPL, provided in the wave interference network to separate a fundamental linearly-polarized, LP, LP01 mode from a LP11 mode into two separate fibers of the wave interference network.

4. The method of any one of claims 1 to 3, further comprising receiving at least one reference signal generated by at least one reference mirror upon reflecting the wave, and generating, via the wave interference network, at least one interference pattern between the scattered signal and the at least one reference signal for acquiring the first signal and the at least one second signal based on the at least one interference pattern.

5. The method of any one of claims 1 to 4, wherein the wave is received from a source via the wave interference network comprising a few-mode fiber network provided as part of a Few-Mode Optical Coherence Tomography, FM-OCT, imaging setup or as part of a laser speckle imaging setup.

6. The method of any one of claims 1 to 5, wherein the scattered signal is one of backscattered and forward scattered by the medium.

7. The method of any one of claims 1 to 6, wherein the first correlation and the second correlation are determined for a same time delay.

8. A system (100) for determining a velocity (206) of one or more objects (204) in a medium, the system comprising
a light source (102) configured to emit a wave excitation:
a wave interference network (106) coupled to the light source and configured to:
receive the wave excitation,
generate a first point spread function, PSF, and at least one second PSF distinct from the first PSF, and
output at least one propagation mode of the wave to the medium for illuminating the medium therewith; and
a computing device (116) coupled to the wave interference network and configured to:
collect a scattered signal from the medium,
acquire a first signal having the first PSF associated therewith and at least one second signal having the at least one second PSF associated therewith;
determine a first correlation of at least one of the first signal and the at least one second signal, and a second correlation of at least one of the first signal and the at least one second signal,
determine a ratio between the first correlation and the second correlation, and
determine the velocity of the one or more objects in the medium based on the ratio.

9. The system of claim 8, wherein the computing device is further configured to determine, based on the ratio, a direction of a flow of the one or more objects in the medium.

10. The system of claim 8 or 9, wherein the computing device is configured to generate the first PSF and the at least one second PSF by separating, via the wave interference network, at least one first propagation mode and at least one second propagation mode of the wave, the first PSF **characterized by** the first step fiber propagation mode and the at least one second PSF **characterized by** the at least one second step fiber propagation mode, preferably wherein the wave interference network comprises a modally specific photonic lantern (MSPL) configured to separate a fundamental linearly-polarized, LP, LP01 mode from a LP11 mode into two separate fibers of the wave interference network.

11. The system of any one of claims 8 to 10, wherein the computing device is configured to determine the first correlation and the second correlation for a same time delay.

12. The system of any one of claims 8 to 11, further comprising at least one reference mirror configured to reflect the wave for generating at least one reference signal, further wherein the computing device is configured to generate, via the wave interference network, at least one interference pattern between the scattered signal and the at least one reference signal for acquiring the first signal and the at least one second signal based on the at least one interference pattern.

13. The system of any one of claims 8 to 12, wherein the wave interference network comprises a few-mode fiber network provided as part of a Few-Mode Optical Coherence Tomography, FM-OCT, imaging setup or as part of a laser speckle imaging setup.

14. The system of any one of claims 8 to 13, wherein the light source comprises at least one single-mode port for emitting into the wave interference network the wave excitation comprising single-mode light or at least one multimode port for emitting into the wave interference network the wave excitation comprising multimode light.

15. The system of any one of claims 8 to 14, further comprising a plurality of detectors configured to receive the scattered signal from the medium and to transmit the scattered signal to the computing device.

## Patentansprüche

1. Verfahren (300) zum Bestimmen einer Geschwindigkeit (206) eines oder mehrerer Objekte (204) in einem Medium (104), wobei das Verfahren umfasst:
Einspeisen einer Welle in ein Welleninterferenz-Netzwerk (106);
Erzeugen einer ersten Punktspreizfunktion, PSF, und mindestens einer zweiten PSF, die sich von der ersten PSF unterscheidet;
Ausgeben, über das Welleninterferenz-Netzwerk, mindestens eines Ausbreitungsmodus der Welle an das Medium, um das Medium damit zu beleuchten;
Sammeln, über das Welleninterferenz-Netzwerk, eines gestreuten Signals aus dem Medium;
Erfassen eines ersten Signals, dem die erste PSF zugeordnet ist, und mindestens eines zweiten Signals, dem die mindestens eine zweite PSF zugeordnet ist;
Bestimmen einer ersten Korrelation von mindestens einem des ersten Signals und des mindestens einen zweiten Signals und einer zweiten Korrelation von mindestens einem des ersten Signals und des mindestens einen zweiten Signals;
Bestimmen eines Verhältnisses zwischen der ersten Korrelation und der zweiten Korrelation; und
Bestimmen der Geschwindigkeit des einen oder der mehreren Objekte im Medium basierend auf dem Verhältnis.

2. Verfahren nach Anspruch 1, ferner umfassend das Bestimmen, basierend auf dem Verhältnis, einer Strömungsrichtung des einen oder der mehreren Objekte im Medium.

3. Verfahren nach Anspruch 1 oder 2, wobei das Erzeugen der ersten PSF und der mindestens einen zweiten PSF das Trennen von mindestens einem ersten Ausbreitungsmodus und mindestens einem zweiten Ausbreitungsmodus der Welle über das Welleninterferenz-Netzwerk umfasst, wobei die erste PSF durch den ersten Stufenfaserausbreitungsmodus gekennzeichnet ist und die mindestens eine zweite PSF durch den mindestens einen zweiten Stufenfaserausbreitungsmodus gekennzeichnet ist, wobei der mindestens eine erste Ausbreitungsmodus und der mindestens eine zweite Ausbreitungsmodus vorzugsweise unter Verwendung einer modenspezifischen photonischen Laterne, MSPL, getrennt sind, die im Welleninterferenz-Netzwerk bereitgestellt ist, um einen grundlegenden linear polarisierten, LP, LP01-Modus von einem LP11-Modus in zwei getrennte Fasern des Welleninterferenz-Netzwerks zu trennen.

4. Verfahren nach einem der Ansprüche 1 bis 3, ferner umfassend das Empfangen mindestens eines Referenzsignals, das von mindestens einem Referenzspiegel bei der Reflektion der Welle erzeugt wird, und das Erzeugen, über das Welleninterferenz-Netzwerk, mindestens eines Interferenzmusters zwischen dem gestreuten Signal und dem mindestens einen Referenzsignal zum Erfassen des ersten Signals und dem mindestens einen zweiten Signal basierend auf dem mindestens einen Interferenzmuster.

5. Verfahren nach einem der Ansprüche 1 bis 4, wobei die Welle von einer Quelle über das Welleninterferenz-Netzwerk empfangen wird, das ein Fasernetzwerk mit wenigen Moden umfasst, das als Teil eines Bildgebungsaufbaus für die optische Kohärenztomographie mit wenigen Moden, FM-OCT, oder als Teil eines Laser-Speckle-Bildgebungsaufbaus bereitgestellt wird.

6. Verfahren nach einem der Ansprüche 1 bis 5, wobei es sich bei dem gestreuten Signal um eines handelt, das vom Medium entweder rückgestreut und vorwärtsgestreut wird.

7. Verfahren nach einem der Ansprüche 1 bis 6, wobei die erste Korrelation und die zweite Korrelation für dieselbe Zeitverzögerung bestimmt werden.

8. System (100) zum Bestimmen einer Geschwindigkeit (206) eines oder mehrerer Objekte (204) in einem Medium, wobei das System umfasst:
eine Lichtquelle (102), die so konfiguriert ist, dass sie eine Wellenanregung aussendet;
ein Welleninterferenz-Netzwerk (106), das mit der Lichtquelle gekoppelt und für Folgendes konfiguriert ist:
Empfangen der Wellenanregung,
Erzeugen einer ersten Punktspreizfunktion, PSF, und mindestens einer zweiten PSF, die sich von der ersten PSF unterscheidet, und
Ausgeben mindestens eines Ausbreitungsmodus der Welle an das Medium, um das Medium damit zu beleuchten; und
eine Rechenvorrichtung (116), die mit dem Welleninterferenz-Netzwerk gekoppelt und für Folgendes konfiguriert ist:
Sammeln eines gestreuten Signals aus dem Medium,
Erfassen eines ersten Signals, dem die erste PSF zugeordnet ist, und mindestens eines zweiten Signals, dem die mindestens eine zweite PSF zugeordnet ist;
Bestimmen einer ersten Korrelation von mindestens einem des ersten Signals und des mindestens einen zweiten Signals und einer zweiten Korrelation von mindestens einem des ersten Signals und des mindestens einen zweiten Signals,
Bestimmen eines Verhältnisses zwischen der ersten Korrelation und der zweiten Korrelation, und
Bestimmen der Geschwindigkeit des einen oder der mehreren Objekte im Medium basierend auf dem Verhältnis.

9. System nach Anspruch 8, wobei die Rechenvorrichtung ferner so konfiguriert ist, dass sie basierend auf dem Verhältnis eine Strömungsrichtung des einen oder der mehreren Objekte im Medium bestimmt.

10. System nach Anspruch 8 oder 9, wobei die Rechenvorrichtung so konfiguriert ist, dass sie die erste PSF und die mindestens eine zweite PSF erzeugt, indem sie über das Welleninterferenz-Netzwerk mindestens einen ersten Ausbreitungsmodus und mindestens einen zweiten Ausbreitungsmodus der Welle voneinander trennt, wobei die erste PSF durch den ersten Stufenfaserausbreitungsmodus gekennzeichnet ist und die mindestens eine zweite PSF durch den mindestens einen zweiten Stufenfaserausbreitungsmodus gekennzeichnet ist, wobei das Welleninterferenz-Netzwerk vorzugsweise eine modenspezifische photonische Laterne (MSPL) umfasst, die so konfiguriert ist, dass sie einen grundlegenden linear polarisierten, LP, LP01-Modus von einem LP11-Modus in zwei separate Fasern des Welleninterferenz-Netzwerks trennt.

11. System nach einem der Ansprüche 8 bis 10, wobei die Rechenvorrichtung so konfiguriert ist, dass sie die erste Korrelation und die zweite Korrelation für dieselbe Zeitverzögerung bestimmt.

12. System nach einem der Ansprüche 8 bis 11, ferner umfassend mindestens einen Referenzspiegel, der so konfiguriert ist, dass er die Welle zum Erzeugen von mindestens einem Referenzsignal reflektiert, wobei die Rechenvorrichtung ferner so konfiguriert ist, dass sie über das Welleninterferenz-Netzwerk mindestens ein Interferenzmuster zwischen dem gestreuten Signal und dem mindestens einen Referenzsignal zum Erfassen des ersten Signals und des mindestens einen zweiten Signals basierend auf dem mindestens einen Interferenzmuster erzeugt.

13. Verfahren nach einem der Ansprüche 8 bis 12, wobei das Welleninterferenz-Netzwerk ein Fasernetzwerk mit wenigen Moden ist, das als Teil eines Bildgebungsaufbaus für die optische Kohärenztomographie mit wenigen Moden, FM-OCT, oder als Teil eines Laser-Speckle-Bildgebungsaufbaus bereitgestellt wird.

14. System nach einem der Ansprüche 8 bis 13, wobei die Lichtquelle mindestens einen Einmoden-Anschluss zum Aussenden der Wellenanregung, die Einmoden-Licht umfasst, in das Welleninterferenz-Netzwerk oder mindestens einen Mehrmoden-Anschluss zum Aussenden der Wellenanregung, die Mehrmoden-Licht umfasst, in das Welleninterferenz-Netzwerk umfasst.

15. System nach einem der Ansprüche 8 bis 14, ferner umfassend eine Vielzahl von Detektoren, die so konfiguriert sind, dass sie das gestreute Signal vom Medium empfangen und das gestreute Signal an die Rechenvorrichtung übertragen.

## Revendications

1. Procédé (300) permettant de déterminer une vitesse (206) d'un ou plusieurs objets (204) dans un milieu (104), le procédé comprenant :
l'injection d'une onde dans un réseau d'interférence d'ondes (106) ;
la génération d'une première fonction d'étalement ponctuel, PSF, et d'au moins une deuxième PSF distincte de la première PSF ;
émettre, via le réseau d'interférence d'ondes, au moins un mode de propagation de l'onde vers le milieu afin d'éclairer ce dernier ;
la collecte, via le réseau d'interférence d'ondes, d'un signal diffusé provenant du milieu ;
acquérir un premier signal auquel est associée la première PSF et au moins un deuxième signal auquel est associée l'au moins une deuxième PSF ;
déterminer une première corrélation entre au moins l'un du premier signal et l'au moins un deuxième signal, et une deuxième corrélation entre au moins l'un du premier signal et l'au moins un deuxième signal ;
déterminer un rapport entre la première corrélation et la deuxième corrélation ; et
déterminer la vitesse du ou des objets dans le milieu sur la base de ce rapport.

2. Procédé selon la revendication 1, comprenant en outre la détermination, sur la base dudit rapport, d'une direction d'écoulement de l'un ou plusieurs objets dans le milieu.

3. Procédé selon la revendication 1 ou 2, dans lequel la génération d' e la première PSF et de la au moins une deuxième PSF comprend la séparation, via le réseau d'interférence d'ondes, d'au moins un premier mode de propagation et d'au moins un deuxième mode de propagation de l'onde, la première PSF étant **caractérisée par** le premier mode de propagation en échelon de la fibre et la au moins une deuxième PSF étant **caractérisée par** le au moins un deuxième mode de propagation en échelon de la fibre, de préférence dans lequel le au moins un premier mode de propagation et le au moins un deuxième mode de propagation sont séparés à l'aide d'une lanterne photonique modale spécifique, MSPL, prévue dans le réseau d'interférence d'ondes pour séparer un mode fondamental à polarisation linéaire, LP, LP01, d'un mode LP11 en deux fibres distinctes du réseau d'interférence d'ondes.

4. Procédé selon l'une quelconque des revendications 1 à 3, comprenant en outre la réception d'au moins un signal de référence généré par au moins un miroir de référence lors de la réflexion de l'onde, et la génération, via le réseau d'interférence d'ondes, d'au moins un motif d'interférence entre le signal diffusé et ledit au moins un signal de référence afin d'acquérir le premier signal et ledit au moins un deuxième signal sur la base dudit au moins un motif d'interférence.

5. Procédé selon l'une quelconque des revendications 1 à 4, dans lequel l'onde est reçue depuis une source via le réseau d'interférence d'ondes comprenant un réseau de fibres à modes réduits fourni dans le cadre d'un dispositif d'imagerie par tomographie par cohérence optique à modes réduits, FM-OCT, ou dans le cadre d'un dispositif d'imagerie par granularité laser.

6. Procédé selon l'une quelconque des revendications 1 à 5, dans lequel le signal diffusé est soit un signal rétrodiffusé, soit un signal diffusé vers l'avant par le milieu.

7. Procédé selon l'une quelconque des revendications 1 à 6, dans lequel la première corrélation et la deuxième corrélation sont déterminées pour un même retard temporel.

8. Système (100) destiné à déterminer une vitesse (206) d'un ou plusieurs objets (204) dans un milieu, le système comprenant :
une source lumineuse (102) configurée pour émettre une onde d'excitation ;
un réseau d'interférence d'ondes (106) couplé à la source lumineuse et configuré pour :
recevoir l'onde d'excitation,
générer une première fonction d'étalement ponctuel, PSF, et au moins une deuxième PSF distincte de la première PSF, et
émettre au moins un mode de propagation de l'onde vers le milieu afin d'éclairer ce dernier avec celle-ci ; et
un dispositif informatique (116) couplé au réseau d'interférence d'ondes et configuré pour :
collecter un signal diffusé provenant du milieu,
acquérir un premier signal auquel est associée une première PSF et au moins un deuxième signal auquel est associée au moins une deuxième PSF ;
déterminer une première corrélation entre au moins l'un parmi le premier signal et l'au moins un deuxième signal, et une deuxième corrélation entre au moins l'un parmi le premier signal et l'au moins un deuxième signal,
déterminer un rapport entre la première corrélation et la deuxième corrélation, et
déterminer la vitesse du ou des objets dans le milieu sur la base de ce rapport.

9. Système selon la revendication 8, dans lequel le dispositif de calcul est en outre configuré pour déterminer, sur la base dudit rapport, une direction d'écoulement du ou des objets dans le milieu.

10. Système selon la revendication 8 ou 9, dans lequel le dispositif de calcul est configuré pour générer la première PSF et la au moins une deuxième PSF en séparant, via le réseau d'interférence d'ondes, au moins un premier mode de propagation et au moins un deuxième mode de propagation de l'onde, la première PSF étant **caractérisée par** le premier mode de propagation en échelon de la fibre et la au moins une deuxième PSF étant **caractérisée par** le au moins un deuxième mode de propagation en échelon de la fibre, de préférence le réseau d'interférence d'ondes comprenant une lanterne photonique à spécificité modale (MSPL) configurée pour séparer un mode fondamental à polarisation linéaire, LP, LP01, d'un mode LP11 dans deux fibres distinctes du réseau d'interférence d'ondes.

11. Système selon l'une quelconque des revendications 8 à 10, dans lequel le dispositif de calcul est configuré pour déterminer la première corrélation et la deuxième corrélation pour un même retard temporel.

12. Système selon l'une quelconque des revendications 8 à 11, comprenant en outre au moins un miroir de référence configuré pour réfléchir l' d'onde afin de générer au moins un signal de référence, dans lequel, en outre, le dispositif de calcul est configuré pour générer, via le réseau d'interférence d'ondes, au moins un motif d'interférence entre le signal diffusé et ledit au moins un signal de référence afin d'acquérir le premier signal et le au moins un deuxième signal sur la base dudit au moins un motif d'interférence.

13. Système selon l'une quelconque des revendications 8 à 12, dans lequel le réseau d'interférence d'ondes comprend un réseau de fibres à modes réduits fourni dans le cadre d'un dispositif d'imagerie par tomographie par cohérence optique à modes réduits, FM-OCT, ou dans le cadre d'un dispositif d'imagerie par granularité laser.

14. Système selon l'une quelconque des revendications 8 à 13, dans lequel la source lumineuse comprend au moins un port à mode unique destiné à émettre dans le réseau d'interférence d'ondes, l'excitation d'onde comprenant une lumière à mode unique ou au moins un port multimode destiné à émettre dans le réseau d'interférence d'ondes l'excitation d'onde comprenant une lumière multimode.

15. Système selon l'une quelconque des revendications 8 à 14, comprenant en outre une pluralité de détecteurs configurés pour recevoir le signal diffusé provenant du milieu et pour transmettre le signal diffusé au dispositif de calcul.
